(19) Europäisches Patentamt
European Patent Office
Office européen des brevets

(11) Veröffentlichungsnummer: **0 588 183 A1**

# (12) EUROPÄISCHE PATENTANMELDUNG

(21) Anmeldenummer: **93114134.5**

(22) Anmeldetag: **03.09.93**

(51) Int. Cl.⁵: **C07D 277/68**, A01N 43/78, C07D 277/76, C07D 417/12

(30) Priorität: **16.09.92 DE 4230903**

(43) Veröffentlichungstag der Anmeldung: **23.03.94 Patentblatt 94/12**

(84) Benannte Vertragsstaaten: **BE CH DE ES FR GB IT LI NL**

(71) Anmelder: **BAYER AG**
**D-51368 Leverkusen(DE)**

(72) Erfinder: **Förster, Heinz, Dr.**
**Am Eckbusch 47**
**D-42113 Wuppertal(DE)**
Erfinder: **Wagner, Klaus, Dr.**
**Jakob-Böhme-Strasse 2**
**D-51065 Köln(DE)**
Erfinder: **Santel, Hans-Joachim, Dr.**
**Grünstrasse 9a**
**D-51371 Leverkusen(DE)**
Erfinder: **Lürssen, Klaus, Dr.**
**August-Kierspel-Strasse 145**
**D-51469 Bergisch Gladbach(DE)**
Erfinder: **Schmidt, Robert R., Dr.**
**Im Waldwinkel 110**
**D-51467 Bergisch Gladbach(DE)**

## (54) Fluorbenzthiazolyloxyacetamide.

(57) Die Erfindung betrifft neue Fluorbenzthiazolyloxyacetamide der Formel (I),

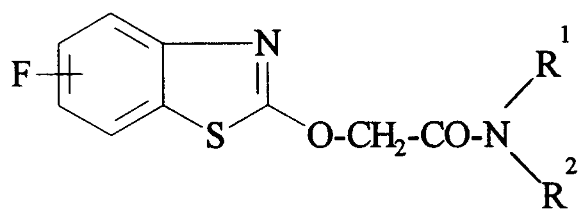

(I)

in welcher

$R^1$ für Wasserstoff oder für einen gegebenenfalls substituierten Rest aus der Reihe Alkyl, Alkenyl, Alkinyl oder Aralkyl steht,

$R^2$ für einen gegebenenfalls substituierten Rest aus der Reihe Alkyl, Alkenyl, Alkinyl, Cycloalkyl, Cycloalkenyl, Aralkyl, Aryl, Alkoxy, Alkenyloxy oder Alkinyloxy steht, oder

$R^1$ und $R^2$ zusammen mit dem Stickstoffatom, an welches sie gebunden sind, einen gegebenenfalls substituierten, gesättigten oder ungesättigten Stickstoff-Heterocyclus bilden, der weitere Heteroatome enthalten kann und an den eine Benzo-Gruppierung anelliert sein kann,

ein Verfahren und neue Zwischenprodukte zu ihrer Herstellung sowie ihre Verwendung als Herbizide.

EP 0 588 183 A1

Die Erfindung betrifft neue Fluorbenzthiazolyloxyacetamide, ein Verfahren und neue Zwischenprodukte zu ihrer Herstellung sowie ihre Verwendung als Herbizide.

Es ist bereits bekannt, daß bestimmte Benzthiazolyloxyacetamide, wie z.B. Benzthiazolyloxyessigsäure-N-methyl-anilid, herbizide Eigenschaften aufweisen (vgl. EP-A-5501; US-A-4 509 971, US-A-4 784 682 und US-A-4 833 243). Die herbizide Wirksamkeit dieser bekannten Verbindungen ist jedoch nicht immer ganz zufriedenstellend.

Es wurden nun neue Fluorbenzthiazolyloxyacetamide der allgemeinen Formel (I) gefunden,

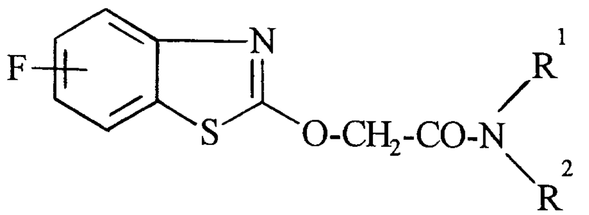

(I)

in welcher

- $R^1$ für Wasserstoff oder für einen gegebenenfalls substituierten Rest aus der Reihe Alkyl, Alkenyl, Alkinyl oder Aralkyl steht,
- $R^2$ für einen gegebenenfalls substituierten Rest aus der Reihe Alkyl, Alkenyl, Alkinyl, Cycloalkyl, Cycloalkenyl, Aralkyl, Aryl, Alkoxy, Alkenyloxy oder Alkinyloxy steht, oder
- $R^1$ und $R^2$ zusammen mit dem Stickstoffatom, an welches sie gebunden sind, einen gegebenenfalls substituierten, gesättigten oder ungesättigten Stickstoff-Heterocyclus bilden, der weitere Heteroatome enthalten kann und an den eine Benzo-Gruppierung anelliert sein kann.

Weiter wurde gefunden, daß man die neuen Fluorbenzthiazolyloxyacetamide der Formel (I) erhält, wenn man Fluorbenzthiazole der allgemeinen Formel (II)

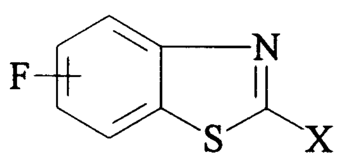

(II)

in welcher

X für Halogen oder Alkylsulfonyl steht,

mit Hydroxyacetamiden der allgemeinen Formel (III)

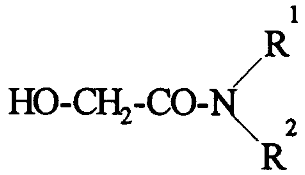

(III)

in welcher

$R^1$ und $R^2$ die oben angegebenen Bedeutungen haben,

gegebenenfalls in Gegenwart eines Verdünnungsmittels, gegebenenfalls in Gegenwart eines Säurebindemittels und gegebenenfalls in Gegenwart eines Katalysators umsetzt.

Schließlich wurde gefunden, daß die neuen Fluorbenzthiazolyloxyacetamide der allgemeinen Formel (I) interessante herbizide Eigenschaften besitzen.

Überraschenderweise zeigen die erfindungsgemäßen Verbindungen der Formel (I) bei teilweise sehr guter Verträglichkeit gegenüber Kulturpflanzen, wie z.B. Reis, erheblich stärkere Wirkung gegen schwer bekämpfbare Unkräuter, wie z.B. Hühnerhirse (Echinochloa crus galli), als die chemisch ähnliche bekannte Verbindung Benzthiazolyloxyessigsäure-N-methyl-anilid.

Gegenstand der Erfindung sind vorzugsweise Verbindungen der Formel (I), in welcher

$R^1$ für Wasserstoff $C_1$-$C_8$-Alkyl (welches gegebenenfalls durch Fluor, Chlor, Cyano oder $C_1$-$C_4$-Alkoxy substituiert ist), für $C_2$-$C_8$-Alkenyl (welches gegebenenfalls durch Fluor und/oder Chlor substituiert ist), für $C_2$-$C_8$-Alkinyl oder für Benzyl steht,

$R^2$ für $C_1$-$C_8$-Alkyl (welches gegebenenfalls durch Fluor, Chlor, Cyano oder $C_1$-$C_4$-Alkoxy substituiert ist), $C_2$-$C_8$-Alkenyl (welches gegebenenfalls durch Fluor und/oder Chlor substituiert ist), für $C_2$-$C_8$-Alkinyl, für $C_3$-$C_6$-Cycloalkyl (welches gegebenenfalls durch Chlor und/oder $C_1$-$C_3$-Alkyl substituiert ist), für $C_5$- oder $C_6$-Cycloalkenyl, für Benzyl (welches gegebenenfalls durch Fluor, Chlor und/oder $C_1$-$C_4$-Alkyl substituiert ist), für Phenyl (welches gegebenenfalls durch Fluor, Chlor, Brom, Iod, Cyano, Nitro, $C_1$-$C_4$-Alkyl, Trifluormethyl, $C_1$-$C_4$-Alkoxy und/oder $C_1$-$C_4$-Alkylthio substituiert ist), für $C_1$-$C_8$-Alkoxy (welches gegebenenfalls durch $C_1$-$C_4$-Alkoxy substituiert ist), oder für $C_3$-$C_4$-Alkenyloxy steht, oder

$R^1$ und $R^2$ zusammen mit dem Stickstoffatom, an das sie gebunden sind, einen gegebenenfalls ein- bis dreifach durch $C_1$-$C_3$-Alkyl substituierten, gesättigten oder ungesättigten fünf- bis siebengliedrigen Stickstoffheterocyclus bilden, welcher gegebenenfalls benzanelliert ist.

Die Erfindung betrifft insbesondere Verbindungen der Formel (I), in welcher

$R^1$ für $C_1$-$C_4$-Alkyl, Allyl oder Propargyl steht,

$R^2$ für $C_1$-$C_6$-Alkyl, $C_1$-$C_2$-Alkoxy-$C_1$-$C_2$-alkyl, Allyl, Propargyl, Cyclopentyl, Cyclohexyl, Cyclohexenyl, Benzyl, Phenyl (welches gegebenenfalls durch Fluor, Chlor, Brom, Cyano, Nitro, Methyl, Ethyl, Trifluormethyl, Methoxy oder Ethoxy substituiert ist), $C_1$-$C_6$-Alkoxy oder $C_1$-$C_2$-Alkoxy-$C_1$-$C_2$-alkoxy steht, oder

$R^1$ und $R^2$ zusammen mit dem Stickstoffatom, an das sie gebunden sind, für gegebenenfalls ein- bis dreifach durch Methyl und/oder Ethyl substituiertes Piperidinyl, für gegebenenfalls ein- oder zweifach durch Methyl und/oder Ethyl substituiertes Pyrrolidinyl, für Perhydroazepinyl oder für 1,2,3,4-Tetrahydro(iso)-chinolinyl stehen.

Ganz besonders bevorzugte Gruppen von Verbindungen der Formel (I) werden nachstehend durch die Formeln (IA), (IB), (IC) und (ID) wiedergegeben, wobei $R^1$ und $R^2$ die oben als bevorzugt bzw. als insbesondere bevorzugt angegebenen Bedeutungen haben.

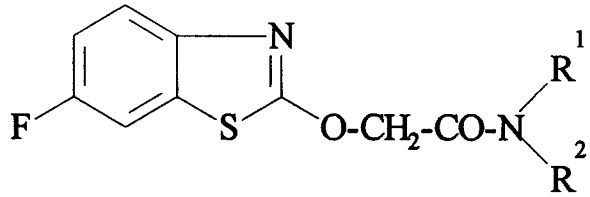

(IA)

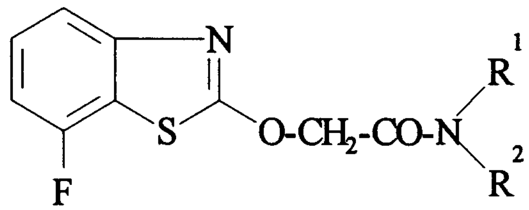

(IB)

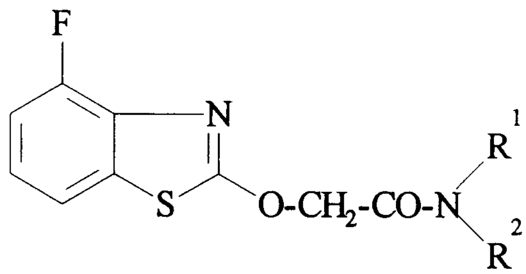

(IC)

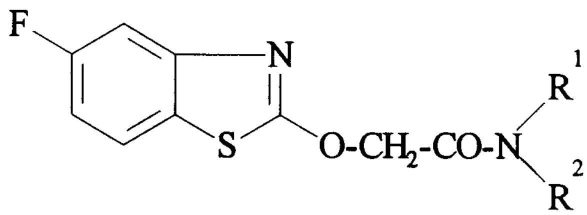

(ID)

Die oben aufgeführten allgemeinen oder in Vorzugsbereichen aufgeführten Restedefinitionen bzw. Erläuterungen gelten für die Endprodukte und für die Ausgangs- und Zwischenprodukte entsprechend. Diese Restedefinitionen können untereinander, also auch zwischen den jeweiligen Vorzugsbereichen beliebig kombiniert werden.

Beispiele für die möglichen Bedeutungen der Gruppierung

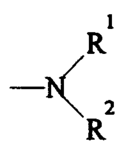

in den Formeln (I), (IA), (IB), (IC) und (ID) sind in der nachstehenden Tabelle 1 aufgeführt.

Tabelle 1: Beispiele für die Bedeutung der Gruppierung

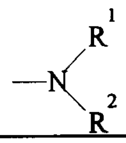

| 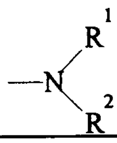 | 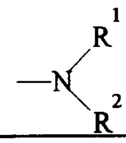 |
|---|---|
| $-N(CH_3)_2$ | $-N(C_4H_9)_2$ |
| $-N(C_2H_5)_2$ | $-N(CH_2CH{=}CH_2)_2$ |
| $-N(C_3H_7)_2$ | $-N(CH_2C{\equiv}CH)_2$ |
| 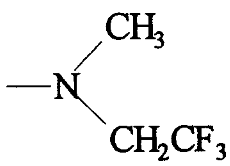 | 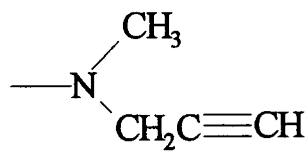 |
| 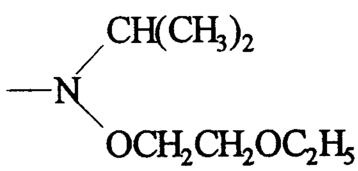 | 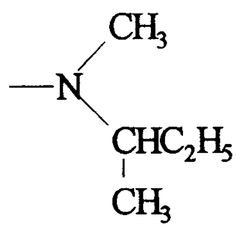 |
| 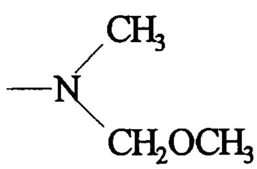 | 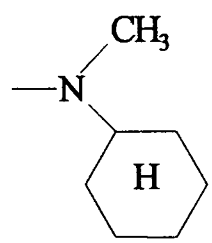 |

Tabelle 1 (Fortsetzung)

| 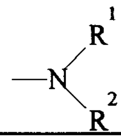 | 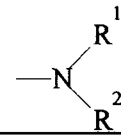 |
|---|---|
| 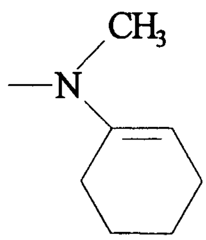 | 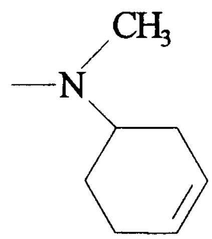 |
| 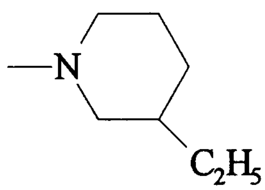 | 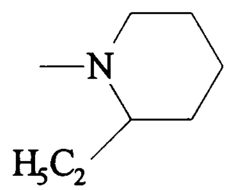 |
| 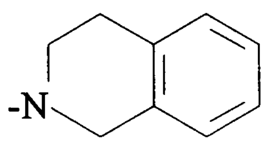 | 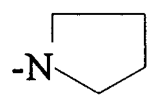 |
| 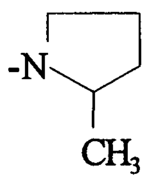 | 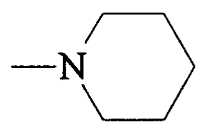 |
| 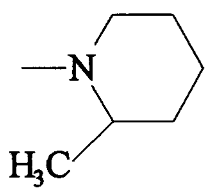 | 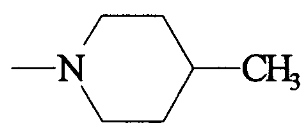 |
| 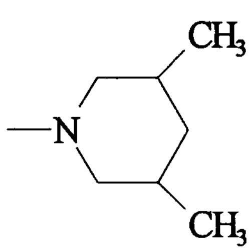 | 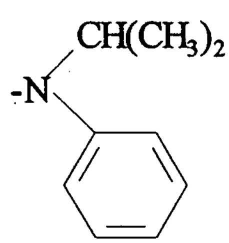 |

Tabelle 1 (Fortsetzung)

| 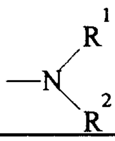 | 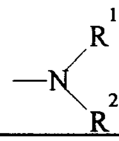 |
|---|---|
| 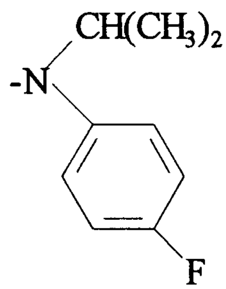 | 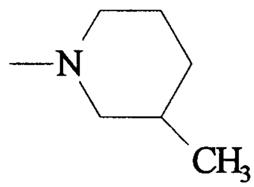 |
| 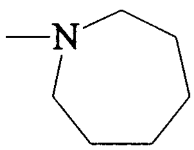 | 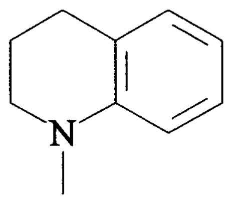 |
| 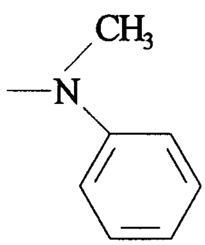 | 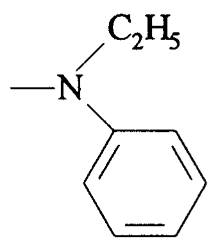 |
| 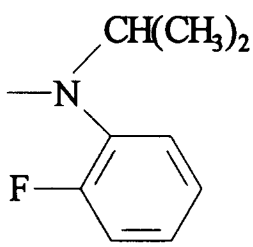 | 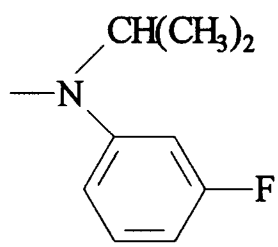 |

Tabelle 1 (Fortsetzung)

| 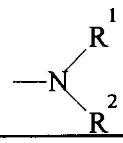 | 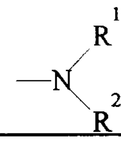 |
| --- | --- |
| 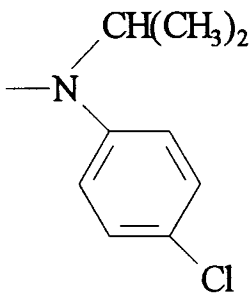 | 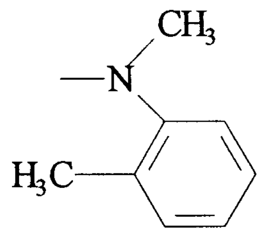 |
| 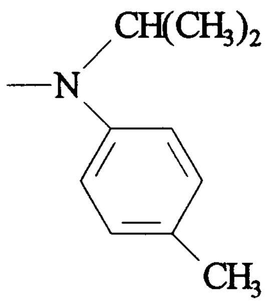 | 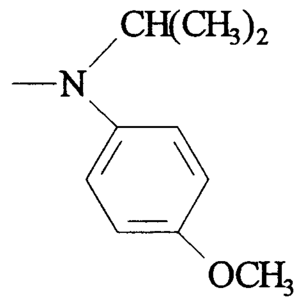 |
| 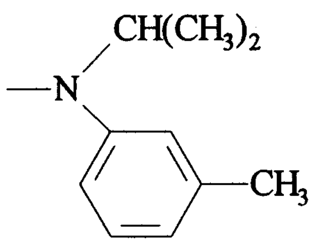 | 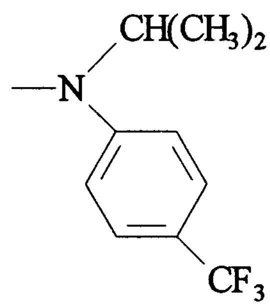 |
| 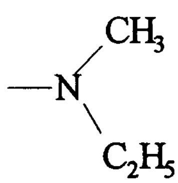 | 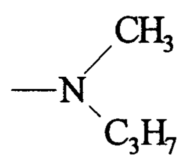 |

Tabelle 1 (Fortsetzung)

| 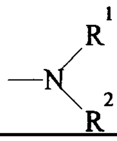 | 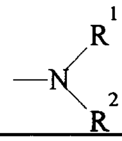 |
|---|---|
| 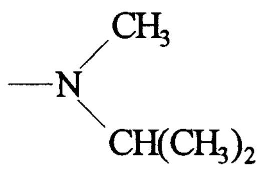 | 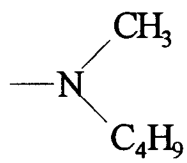 |
| 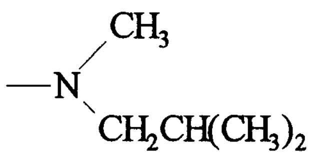 | 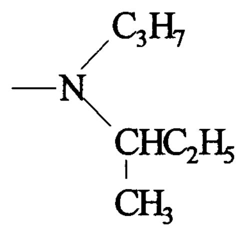 |
| 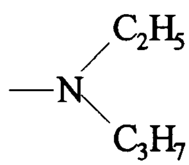 | 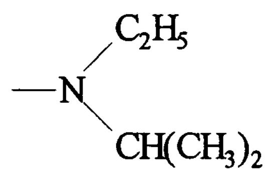 |
| 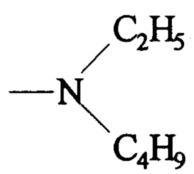 | 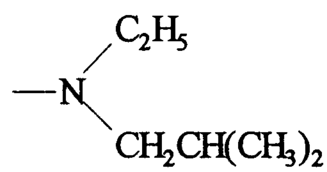 |
| 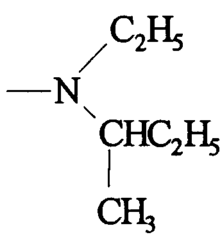 | 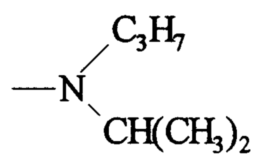 |
| 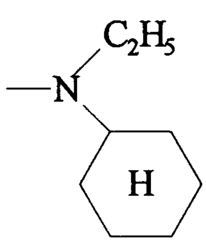 | 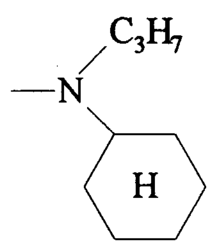 |

Tabelle 1 (Fortsetzung)

| 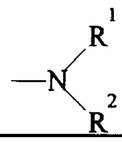 | 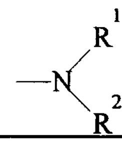 |
|---|---|
| 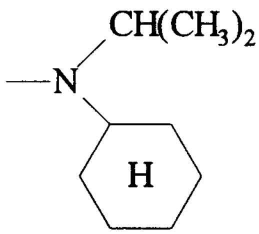 | 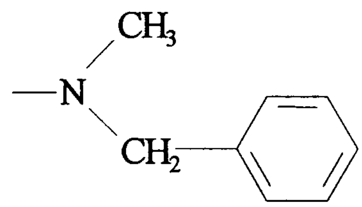 |
| 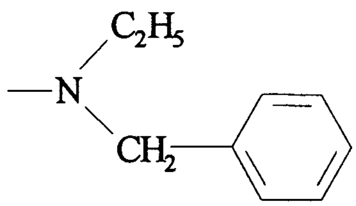 | 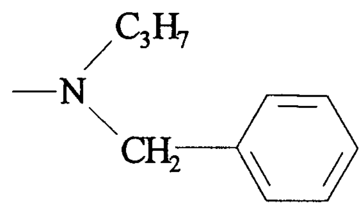 |
| 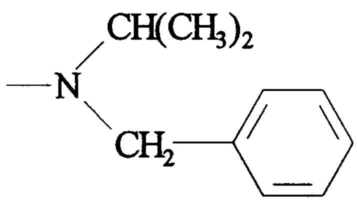 | 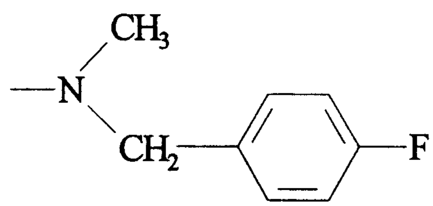 |
| 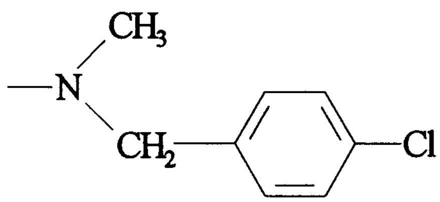 | 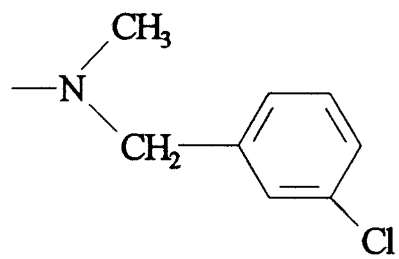 |
| 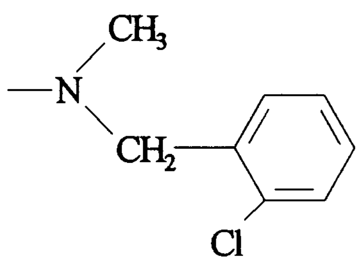 | 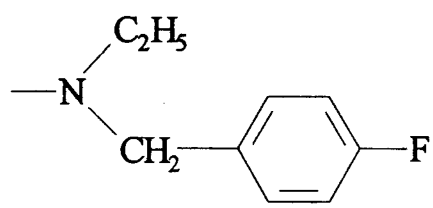 |

Tabelle 1 (Fortsetzung)

| 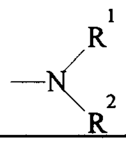 | 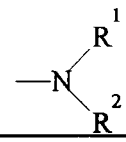 |
| --- | --- |
| 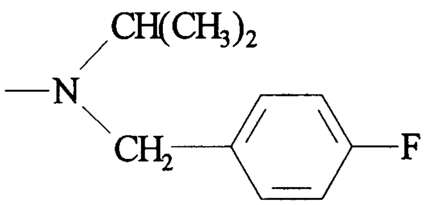 | 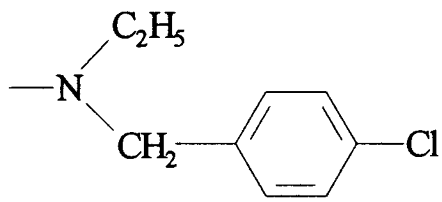 |
| 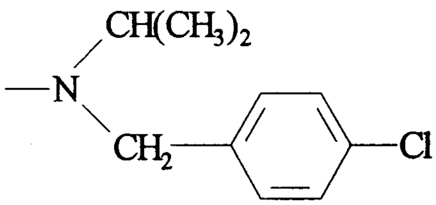 | 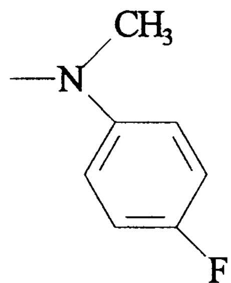 |
| 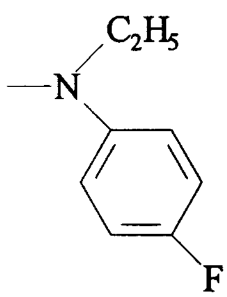 | 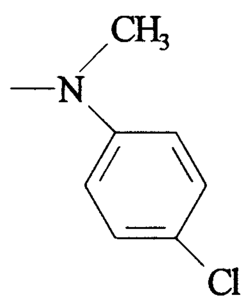 |
| 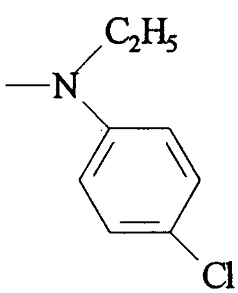 | 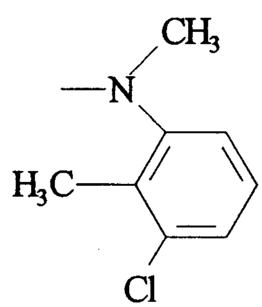 |

Tabelle 1 (Fortsetzung)

| 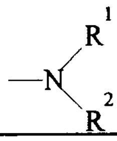 | 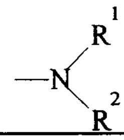 |
|---|---|
| 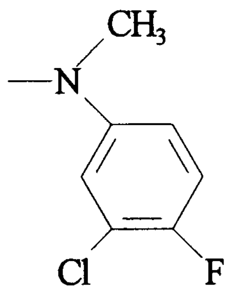 | 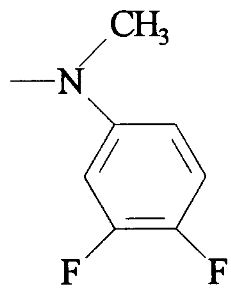 |
| 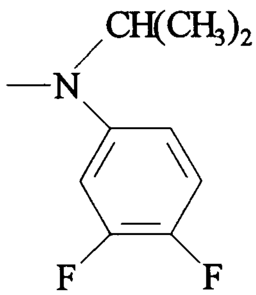 | 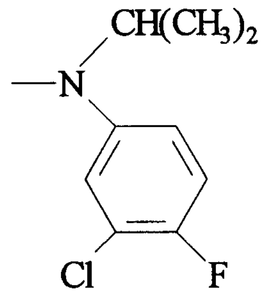 |
| 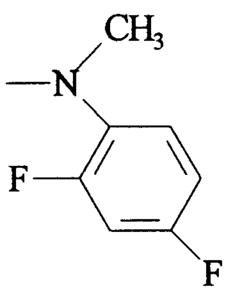 | 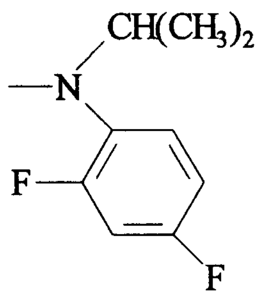 |
| 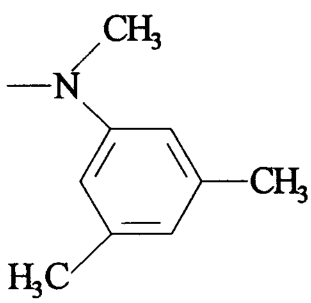 | 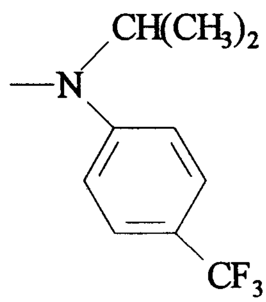 |

Tabelle 1 (Fortsetzung)

| 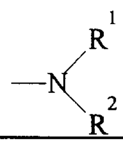 | 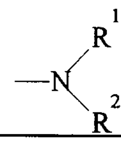 |
|---|---|
| 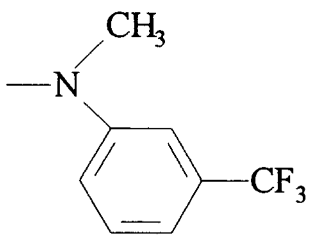 | 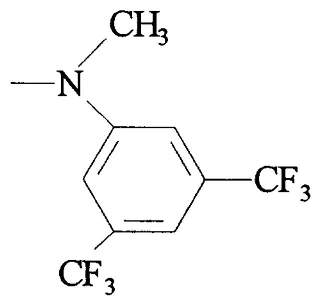 |
| 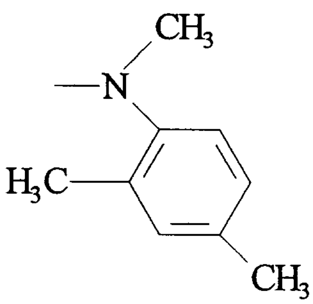 | 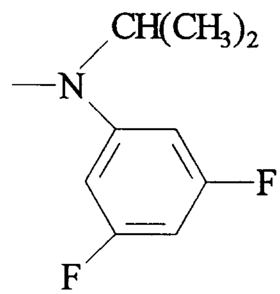 |
| 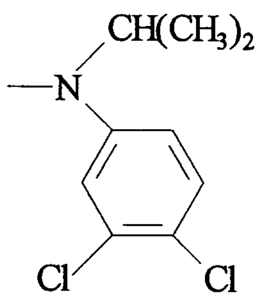 | 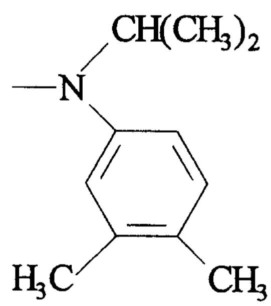 |
| 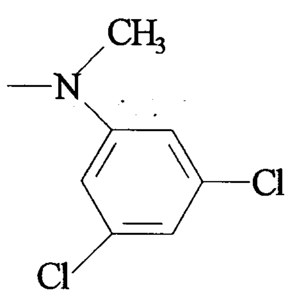 | 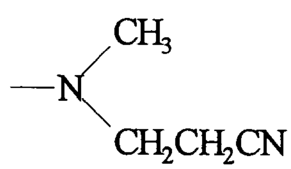 |

Tabelle 1 (Fortsetzung)

| 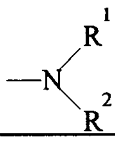 | 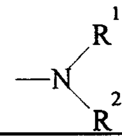 |
|---|---|
| 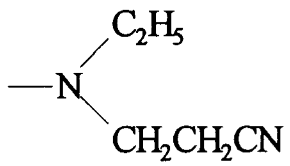 | $-N(CH_2CH_2CN)_2$ |
| 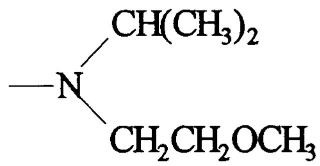 | 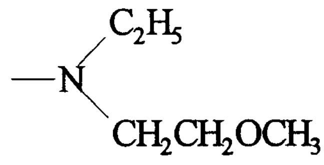 |
| 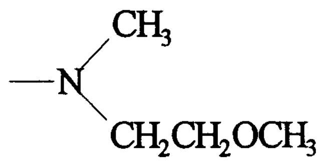 | 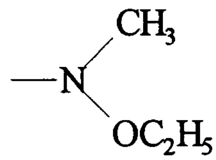 |
| 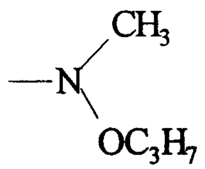 | 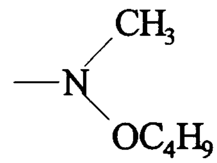 |
| 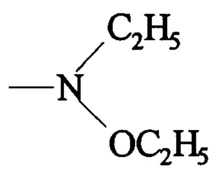 | 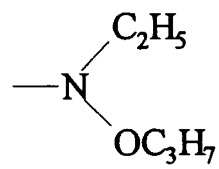 |
| 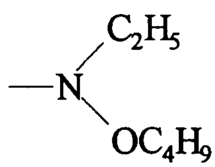 | 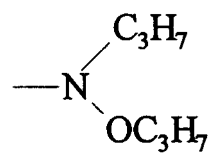 |
| 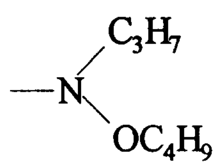 | 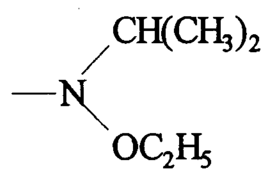 |

Tabelle 1 (Fortsetzung)

| 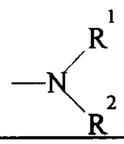 | 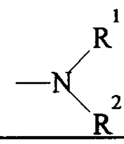 |
|---|---|
| 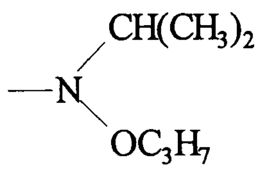 | 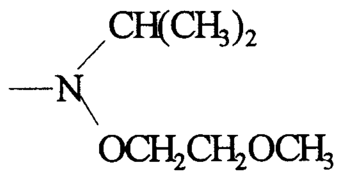 |
| 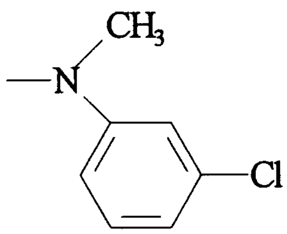 | 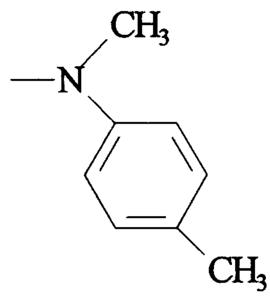 |
| 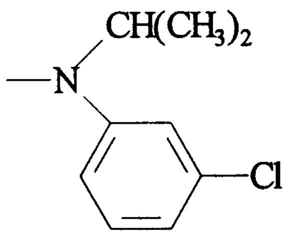 | 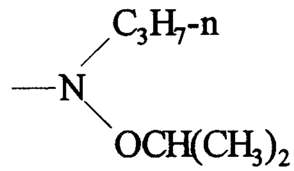 |
| 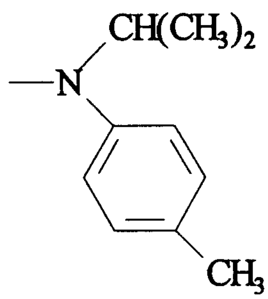 | |

Verwendet man beispielsweise 2-Chlor-6-fluor-benzthiazol und N,N-Diethyl-hydroxyacetamid als Ausgangsstoffe, so läßt sich der Reaktionsablauf beim erfindungsgemäßen Herstellungsverfahren durch das folgende Formelschema darstellen:

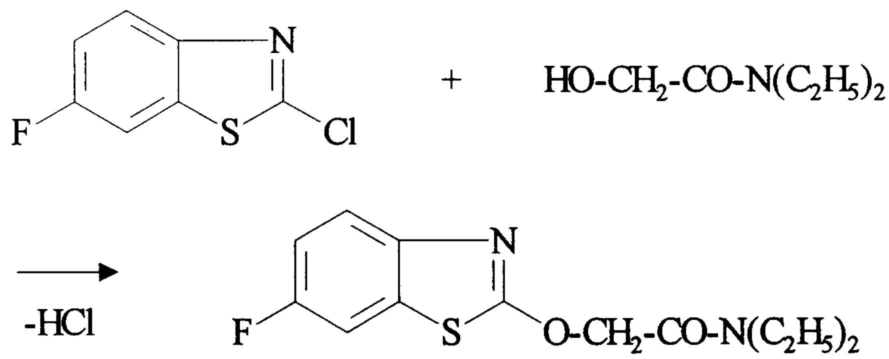


Die beim erfindungsgemäßen Verfahren zur Herstellung der Verbindungen der Formel (I) als Ausgangsstoffe zu verwendenden Fluorbenzthiazole sind durch die Formel (II) allgemein definiert.

In der Formel (II) steht X vorzugsweise für Fluor, Chlor oder Brom sowie für $C_1$-$C_4$-Alkylsulfonyl, insbesondere für Chlor oder Methylsulfonyl.

Die Ausgangsstoffe der Formel (II) sind mit Ausnahme von 2,6-Difluor-benzthiazol und 2-Chlor-6-fluor-benzthiazol (vgl. Org. Magn. Reson. 7 (1975), 84-85 - zitiert in Chem. Abstracts 83:113186x) noch nicht aus der Literatur bekannt und sind mit Ausnahme der beiden genannten Verbindungen als neue Stoffe ebenfalls Gegenstand der vorliegenden Erfindung.

Als Beispiele für die neuen Verbindungen der Formel (II) seien genannt:
2-Chlor-4-fluor-benzthiazol, 2-Chlor-5-fluor-benzthiazol, 2-Chlor-7-fluor-benzthiazol, 2-Methylsulfonyl-4-fluor-benzthiazol, 2-Methylsulfonyl-5-fluor-benzthiazol, 2-Methylsulfonyl-6-fluor-benzthiazol und 2-Methylsulfonyl-7-fluor-benzthiazol.

Man erhält die Fluorbenzthiazole der allgemeinen Formel (II), wenn man

(a) für den Fall, daß X für Halogen steht,

2-Amino-fluorbenzthiazole der allgemeinen Formel (IV)

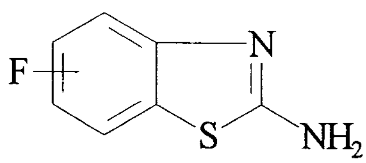
(IV)

mit Diazotierungs- und Halogenierungsmitteln, wie z.B. Natriumnitrit, Salzsäure und Kupferpulver, bei Temperaturen zwischen -10 °C und +60 °C umsetzt (vgl. die Herstellungsbeispiele).

Die 2-Amino-fluorbenzthiazole der Formel (IV) sind bekannt und/oder können nach an sich bekannten Verfahren hergestellt werden (vgl. J. Chem. Soc. C 1969, 268-272; J. Heterocycl. Chem. 18 (1981), 759-761; Synthet. Commun. 17 (1987), 229-240; EP-A 282971; Herstellungsbeispiele).

Man erhält die Fluorbenzthiazole der Formel (II) auch, wenn man

(b) für den Fall, daß X für Halogen steht,

2-Mercapto-fluorbenzthiazole der allgemeinen Formel (V)

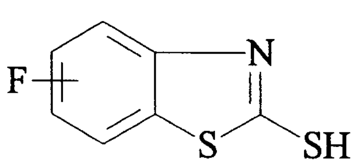
(V)

mit Halogenierungsmitteln, wie z.B. Thionylchlorid, gegebenenfalls in Gegenwart von Reaktionshilfsmitteln, wie z.B. Dimethylformamid, bei Temperaturen zwischen 0 °C und 100 °C umsetzt (vgl. die Herstellungsbeispiele),

oder wenn man

(c) für den Fall, daß X für Alkylsulfonyl steht,
2-Mercapto-fluorbenzthiazole der Formel (V) mit Alkylierungsmitteln, wie z.B. Methyliodid, gegebenenfalls in Gegenwart von Säurebindemitteln, wie z.B. Natriumhydroxid, und in Gegenwart von Verdünnungsmitteln, wie z.B. Wasser, bei Temperaturen zwischen -10 °C und +100 °C umsetzt und die so erhaltenen Alkylierungsprodukte mit Oxidationsmitteln, wie z.B. Hydrogenperoxid, gegebenenfalls in Gegenwart von Reaktionshilfsmitteln, wie z.B. Ameisensäure, Schwefelsäure und Ammoniummolybdat, bei Temperaturen zwischen 0 °C und 50 °C umsetzt (vgl. die Herstellungsbeispiele).

Die 2-Mercapto-fluorbenzthiazole der Formel (V) sind bekannt und/oder können nach an sich bekannten Verfahren hergestellt werden (vgl. DE-OS 3 008 225; US-P-4 873 346; Herstellungsbeispiele).

Die beim erfindungsgemäßen Verfahren zur Herstellung von Verbindungen der Formel (I) weiter als Ausgangsstoffe zu verwendenden Hydroxyessigsäureamide sind durch die Formel (III) allgemein definiert.

In Formel (III) haben $R^1$ und $R^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$ und $R^2$ angegeben wurden.

Die Hydroxyessigsäureamide der Formel (III) sind bekannt und/oder können nach an sich bekannten Verfahren hergestellt werden (vgl. US-P-4 509 971 und US-P-4 645 525; ferner US-P-4 334 073, DE-OS 3 038 598, DE-OS 3 038 636, EP-A-37 526, EP-A-348 737, DE-OS 3 819 477).

Das erfindungsgemäße Verfahren zur Herstellung der neuen Fluorbenzthiazolyloxyacetamide der Formel (I) wird vorzugsweise unter Verwendung von Verdünnungsmitteln durchgeführt. Hierzu gehören vorzugsweise Kohlenwasserstoffe, wie z.B. Toluol, Xylol oder Cyclohexan, Halogenkohlenwasserstoffe, wie z.B. Methylenchlorid, Ethylenchlorid, Chloroform oder Chlorbenzol, Ether, wie z.B. Diethylether, Dipropylether, Diisopropylether, Dibutylether, Diisobutylether, Glycoldimethylether, Tetrahydrofuran und Dioxan, Alkohole, wie z.B. Methanol, Ethanol, Propanol, Isopropanol, Butanol, Isobutanol, sec-Butanol oder tert-Butanol, Ketone, wie z.B. Aceton, Methylethylketon, Methylisopropylketon und Methylisobutylketon, Ester, wie z.B. Essigsäuremethylester und Essigsäureethylester, Amide, wie z.B. Dimethylformamid, Dimethylacetamid und N-Methyl-pyrrolidon, Nitrile, wie z.B. Acetonitril und Propionitril, Sulfoxide, wie z.B. Dimethylsulfoxid sowie Wasser oder wäßrige Salzlösungen.

Als Salze verwendet man hierbei vorzugsweise Chloride oder Sulfate von Alkali- oder Erdalkalimetallen, wie beispielsweise Natriumchlorid, Kaliumchlorid oder Calciumchlorid. Besonders bevorzugt ist Natriumchlorid.

Das erfindungsgemäße Verfahren wird vorteilhaft unter Verwendung von Säurebindemitteln durchgeführt. Als solche werden vorzugsweise stark basische Alkali- und Erdalkalimetallverbindungen, beispielsweise Oxide, wie z.B. Natrium-, Kalium-, Magnesium- und Calciumoxid, Hydroxide, wie z.B. Natrium-, Kalium-, Magnesium- und Calciumhydroxid, Alkoholate, wie z.B. Natrium- und Kalium-tert-butylat und/oder Carbonate, wie z.B. Natrium-, Kalium-, Magnesium- und Calciumcarbonat verwendet.

Der Zusatz von 0,01 bis 10 Gew. -% (bezogen auf eingesetztes Glycolsäureamid der Formel (III)) eines Phasentransferkatalysators mag sich in einigen Fällen als vorteilhaft erweisen. Als Beispiele für solche Katalysatoren seien genannt:

Tetrabutylammoniumchlorid, Tetrabutylammoniumbromid, Tributyl-methylphosphoniumbromid, Trimethyl-$C_{13}/C_{15}$-alkyl-ammoniumchlorid, Dibenzyl-dimethyl-ammonium-methylsultat, Dimethyl-$C_{12}/C_{14}$-alkylbenzylammoniumchlorid, Tetrabutylammoniumhydroxid, 18-Krone-6, Triethylbenzylammoniumchlorid, Trimethylbenzylammoniumchlorid, Tetraethylammoniumbromid.

Die Reaktionstemperaturen können bei dem erfindungsgemäßen Verfahren in einem größeren Bereich variiert werden. Im allgemeinen arbeitet man bei Temperaturen zwischen -50 °C und +110 °C, vorzugsweise bei Temperaturen zwischen -20 °C und +80 °C.

Das erfindungsgemäße Verfahren wird im allgemeinen bei Normaldruck durchgeführt, es kann aber auch bei erhöhtem oder vermindertem Druck, etwa zwischen 0,1 und 10 bar, durchgeführt werden.

Zur Durchführung des erfindungsgemäßen Verfahrens setzt man je Mol Fluorbenzthiazol der Formel (II) im allgemeinen 0,5 bis 5 Mol, vorzugsweise 0,8 bis 1,5 Mol, Hydroxyessigsäureamid der Formel (III) ein. Die Reaktionskomponenten können in beliebiger Reihenfolge zusammengegeben werden. Man rührt jeweils das Reaktionsgemisch bis zum Ende der Umsetzung und arbeitet nach üblichen Methoden auf (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden.Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga,

Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Urkrautbekämpfung in Dauerkulturen, z.B. Forst, Ziergehölz-, Obst-, Wein-, Zitrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen Unkräutern, wie z.B. Hühnerhirse (Echinochloa Crus Galli) in verpflanztem Reis. Sie können aber auch allgemein zur Bekämpfung monokotyler Unkräuter in dikotylen Kulturpflanzenbeständen eingesetzt werden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel könne z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser.

Als feste Trägerstoffe kommen infrage: z.B. Ammoniumsalze und natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder

Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl, Azinone, wie z.B. Chloridazon und Norflurazon; Carbamate, wie z.B. Chlorpropharn, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und loxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamte, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithlopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen; Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro Hektar.

Die Herstellung und die Verwendung der erfindungsgemäßen Wirkstoffe geht aus den nachfolgenden Beispielen hervor.

Herstellungsbeispiele:

Beispiel 1

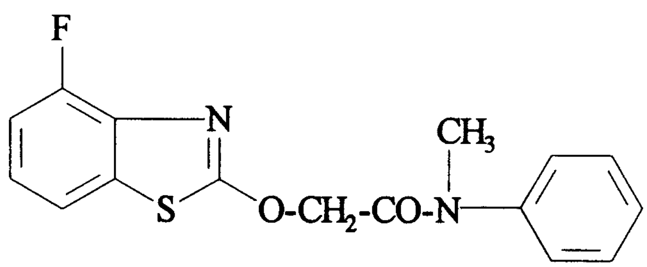

Eine Lösung von 0,6 g (15 mMol) Natriumhydroxid in 2,7 ml Wasser wird bei 20°C unter Rühren tropfenweise zu einer Mischung aus 2,5 g (15 mMol) N-Methyl-hydroxyacetanilid, 3,5 g (15 mMol) 2-Methylsulfonyl-4-fluor-benzthiazol und 50 ml Aceton gegeben. Das Reaktionsgemisch wird 12 Stunden bei 20°C gerührt und dann auf etwa das doppelte Volumen Wasser gegossen. Das hierbei kristallin angefallene Produkt wird durch Absaugen isoliert.

Man erhält 3,8 g (79% der Theorie) N-Methyl-α-(4-fluor-benzthiazol-2-yl-oxy)-acetanilid vom Schmelzpunkt 94°C.

Beispiel 2

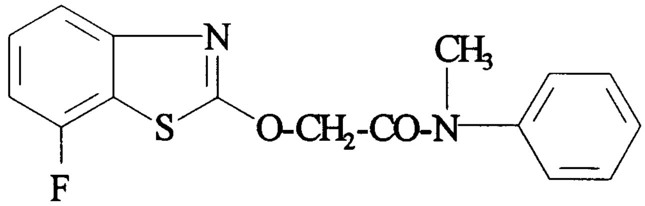

Eine Lösung von 3,75 g (16,6 mMol) 2-Chlor-7-fluor-benzthiazol in 20 ml Acetonitril wird unter Rühren tropfenweise zu einer auf -10 °C abgekühlten Mischung aus 2,74 g (16,6 mMol) N-Methyl-hydroxyacetanilid, 0,93 g (16,6 mMol) Kaliumhydroxid und 50 ml Isopropanol gegeben. Das Reaktionsgemisch wird 6 Stunden bei 0 °C bis 5 °C gerührt, dann auf etwa das doppelte Volumen Wasser gegossen und dreimal mit Methylenchlorid extrahiert. Die vereinigten organischen Phasen werden mit Wasser gewaschen, mit Natriumsulfat getrocknet und filtriert. Nach Einengen des Filtrats wird der Rückstand durch zweimalige Säulenchromatographie gereinigt (1. Kieselgel/Methylenchlorid, 2. Kieselgel/Methyl-tert-butylether).

Man erhält 1,4 g (27% der Theorie) N-Methyl-α-(7-fluor-benzthiazol-2-yl-oxy)-acetanilid vom Schmelzpunkt 116 °C.

Analog zu den Beispielen 1 und 2 sowie entsprechend der allgemeinen Beschreibung des erfindungsgemäßen Herstellungsverfahrens können beispielsweise auch die in der nachstehenden Tabelle 2 aufgeführten Verbindungen der Formel (I) hergestellt werden:

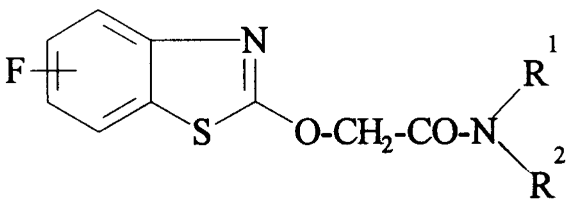

(I)

Tabelle 2: Herstellungsbeispiele für die Verbindungen der Formel (I)

| Bsp.-Nr. | Position von F | $R^1$ | $R^2$ | Schmelzpunkt (°C) (Brechungsindex) |
|---|---|---|---|---|
| 3 | 5 | $CH_3$ | 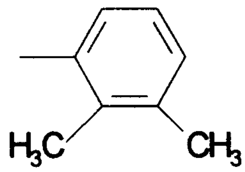 | 144 |
| 4 | 5 | $CH(CH_3)_2$ | 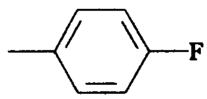 | 115 |
| 5 | 5 | $CH(CH_3)_2$ | 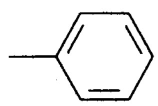 | 113 |
| 6 | 5 | $CH_3$ | 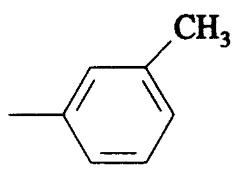 | 115 |
| 7 | 5 | $CH_3$ | 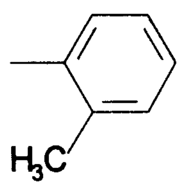 | 146 |

Tabelle 2 (Fortsetzung)

| Bsp.-Nr. | Position von F | $R^1$ | $R^2$ | Schmelzpunkt (°C) (Brechungsindex ) |
|---|---|---|---|---|
| 8 | 5 | $-(CH_2)_6$ | | 114 |
| 9 | 5 | $C_2H_5$ | 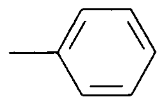 | 97 |
| 10 | 5 | $CH_3$ | $n-C_4H_9$ | 53 |
| 11 | 5 | $-CH_2CH=CH_2$ | $-CH_2CH=CH_2$ | ($n_D^{20}$ : 1,5345) |
| 12 | 5 | $CH_3$ | 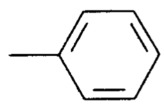 | 92 |
| 13 | 6 | $CH(CH_3)_2$ | 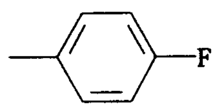 | 72 |
| 14 | 6 | $CH(CH_3)_2$ | 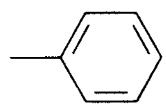 | 108 |
| 15 | 6 | $CH_3$ | 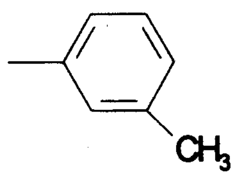 | 83 |
| 16 | 6 | $C_2H_5$ | $C_2H_5$ | 65 |
| 17 | 6 | $-CH(CH_3)-(CH_2)_5-$ | | ($n_D^{20}$ : 1,5510) |
| 18 | 6 | $CH(CH_3)_2$ | $-OC_2H_4OC_2H_5$ | ($n_D^{20}$ : 1,5128) |
| 19 | 6 | $-(CH_2)_6-$ | | 55 |
| 20 | 6 | $CH(CH_3)_2$ | $-OCH(CH_3)_2$ | 46 |

Tabelle 2 (Fortsetzung)

| Bsp.-Nr. | Position von F | $R^1$ | $R^2$ | Schmelzpunkt (°C) (Brechungsindex) |
|---|---|---|---|---|
| 21 | 6 | $-CH_2CH=CH_2$ | $-CH_2CH=CH_2$ | 68 |
| 22 | 6 | $CH_3$ | $-CH(CH_3)-C_2H_5$ | 69 |
| 23 | 6 | ( 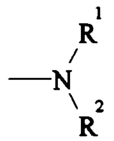 : | 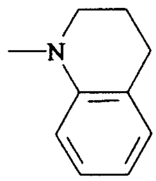 ) | 89 |
| 24 | 6 | $n-C_4H_9$ | $n-C_4H_9$ | ($n_D^{20}$ : 1,5321) |
| 25 | 6 | $CH_3$ | $n-C_4H_9$ | 66 |
| 26 | 6 | $CH(CH_3)_2$ | 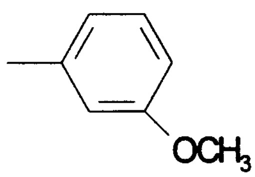 | 86 |
| 27 | 5 | $CH(CH_3)_2$ | 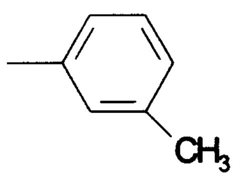 | 125 |
| 28 | 5 | ( 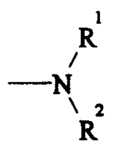 : | 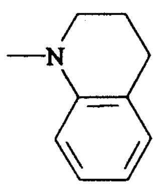 ) | 128 |
| 29 | 7 | $CH(CH_3)_2$ | 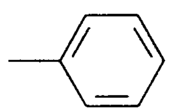 | 107 |
| 30 | 5 | $CH(CH_3)_2$ | $-OCH(CH_3)_2$ | ($n_D^{20}$ : 1,5372) |

Tabelle 2 (Fortsetzung)

| Bsp.-Nr. | Position von F | $R^1$ | $R^2$ | Schmelzpunkt (°C) (Brechungsindex) |
|---|---|---|---|---|
| 31 | 5 | $-CH(CH_3)_2$ | $-OC_2H_4OC_2H_5$ | ($n_D^{20}$: 1,5365) |
| 32 | 5 | $-CH_2-CH(CH_3)-(CH_2)_3-$ | | 64 |
| 33 | 4 | $CH(CH_3)_2$ | 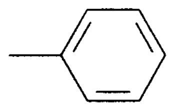 | 92 |
| 34 | 4 | $CH(CH_3)_2$ | 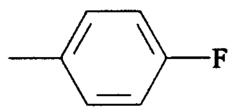 | 76 |
| 35 | 4 | $CH_3$ | 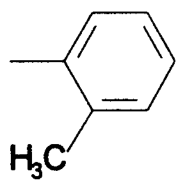 | 145 |
| 36 | 4 | $CH_3$ | 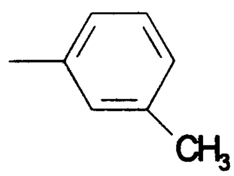 | 86 |
| 37 | 4 | $-CH_2CH=CH_2$ | $-CH_2CH=CH_2$ | 56 |
| 38 | 4 | $-CH(CH_3)_2$ | $-OC_2H_4OC_2H_5$ | ($n_D^{20}$: 1,4991) |
| 39 | 7 | $-CH(CH_3)_2$ | 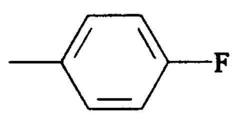 | 108 |
| 40 | 7 | $CH(CH_3)_2$ | $-OC_2H_4OC_2H_5$ | 46 |
| 41 | 7 | $CH(CH_3)_2$ | $-OCH(CH_3)_2$ | 41 |

Tabelle 2 (Fortsetzung)

| Bsp.-Nr. | Position von F | $R^1$ | $R^2$ | Schmelzpunkt (°C) (Brechungsindex) |
|---|---|---|---|---|
| 42 | 7 | $CH_3$ | 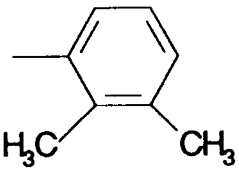 | 121 |
| 43 | 7 | $CH_3$ | 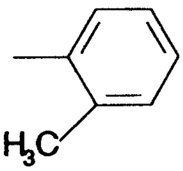 | 104 |
| 44 | 7 | $CH_3$ | $n\text{-}C_4H_9$ | 61 |
| 45 | 7 | $-(CH_2)_6-$ | | 88 |
| 46 | 7 | $CH_3$ | 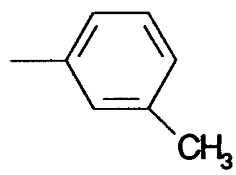 | 99 |
| 47 | 7 | $CH(CH_3)_2$ | 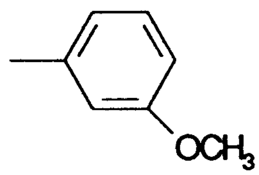 | 113 |
| 48 | 7 | $-CH_2CH{=}CH_2$ | $-CH_2CH{=}CH_2$ | 56 |
| 49 | 5 | $CH_3$ | $-CH(CH_3)\text{-}C_2H_5$ | ($n_D^{20}$: 1,5305) |
| 50 | 5 | $CH(CH_3)_2$ | $n\text{-}C_6H_{13}$ | ($n_D^{20}$: 1,5208) |
| 51 | 5 | $CH(CH_3)_2$ | $-CH_2CH_2CH(CH_3)_2$ | ($n_D^{20}$: 1,5035) |
| 52 | 5 | $C_2H_5$ | $C_2H_5$ | ($n_D^{20}$: 1,5395) |

Tabelle 2 (Fortsetzung)

| Bsp.-Nr. | Position von F | $R^1$ | $R^2$ | Schmelzpunkt (°C) (Brechungsindex) |
|---|---|---|---|---|
| 53 | 5 | $-CH_2-CH(C_2H_5)-(CH_2)_3-$ | | ($n_D^{20}$ : 1,5465 ) |
| 54 | 5 | $CH(CH_3)_2$ | 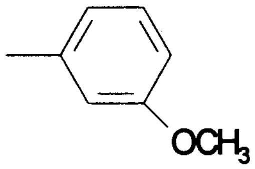 | 102 |
| 55 | 5 | $n-C_4H_9$ | $n-C_4H_9$ | 42 |
| 56 | 5 | $n-C_3H_7$ | $n-C_3H_7$ | 60 |
| 57 | 5 | $-CH(CH_3)-(CH_2)_4-$ | | 96 |
| 58 | 4 | $CH(CH_3)_2$ | 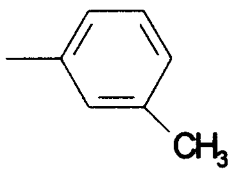 | 138 |
| 59 | 4 | $CH(CH_3)_2$ | $-OCH(CH_3)_2$ | 52 |
| 60 | 4 | $n-C_4H_9$ | $n-C_4H_9$ | 34 |
| 61 | 4 | $-(CH_2)_6-$ | | 75 |
| 62 | 4 | $-CH_2-CH(C_2H_5)-(CH_2)_3-$ | | ($n_D^{20}$ : 1,5032) |
| 63 | 4 | $CH_3$ | $-CH(CH_3)-C_2H_5$ | 72 |

Tabelle 2 (Fortsetzung)

| Bsp.-Nr. | Position von F | $R^1$ | $R^2$ | Schmelzpunkt (°C) (Brechungsindex) |
|---|---|---|---|---|
| 64 | 4 | $n-C_3H_7$ | $n-C_3H_7$ | 49 |
| 65 | 4 | $-CH(CH_3)-(CH_2)_4-$ | | 67 |
| 66 | 4 | $C_2H_5$ | $C_2H_5$ | 73 |
| 67 | 4 | $C_2H_5$ | 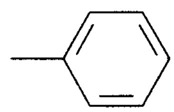 | 100 |
| 68 | 6 | $CH_3$ | 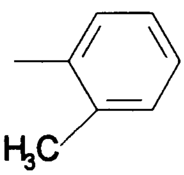 | 113 |
| 69 | 5 | $CH(CH_3)_2$ | $-CH_2-CH(CH_3)-CH(CH_3)-C_2H_5$ | ($n_D^{20}$ : 1,5246) |
| 70 | 5 | $n-C_3H_7$ | $-CH_2CH(C_2H_5)_2$ | ($n_D^{20}$ : 1,5132) |
| 71 | 6 | $CH_3$ | 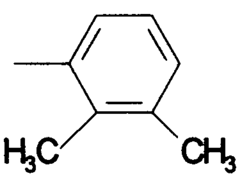 | 148 |
| 72 | 6 | $-CH_2-CH(C_2H_5)-(CH_2)_3-$ | | ($n_D^{20}$ : 1,5088) |
| 73 | 6 | $n-C_3H_7$ | $n-C_3H_7$ | ($n_D^{20}$ : 1,5391) |
| 74 | 7 | $n-C_4H_9$ | $n-C_4H_9$ | ($n_D^{20}$ : 1,5095) |
| 75 | 7 | $CH(CH_3)_2$ | 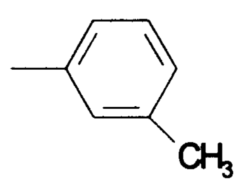 | 84 |
| 76 | 7 | $n-C_3H_7$ | $n-C_3H_7$ | 70 |

Tabelle 2 (Fortsetzung)

| Bsp.- Nr. | Position von F | $R^1$ | $R^2$ | Schmelzpunkt (°C) (Brechungsindex) |
|---|---|---|---|---|
| 77 | 7 | $-CH(CH_3)-(CH_2)_4-$ | | 88 |
| 78 | 7 | $CH_3$ | $-CH(CH_3)-C_2H_5$ | 49 |
| 79 | 7 | $C_2H_5$ | 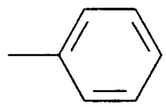 | 92 |
| 80 | 6 | $C_2H_5$ | 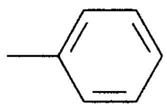 | 107 |
| 81 | 7 | $-CH_2-CH(C_2H_5)-(CH_2)_3-$ | | 60 |
| 82 | 7 | $C_2H_5$ | $C_2H_5$ | 45 |
| 83 | 4 | $CH_3$ | 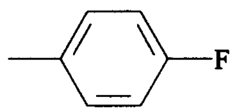 | 84 |
| 84 | 5 | $CH_3$ | 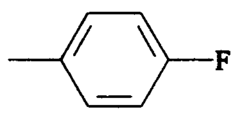 | 85 |
| 85 | 7 | $CH_3$ | 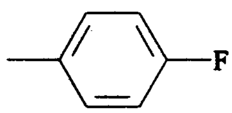 | 96 |
| 86 | 4 | $C_2H_5$ | 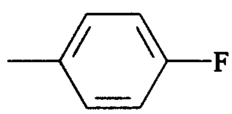 | 107 |
| 87 | 7 | $C_2H_5$ | 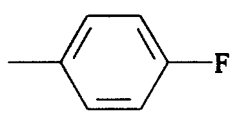 | 119 |
| 88 | 5 | $C_2H_5$ | 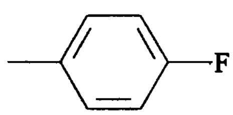 | 116 |

Ausgangsstoffe der Formel (II):

Beispiel (II-1)

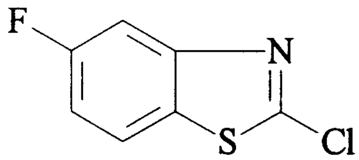

Stufe 1:

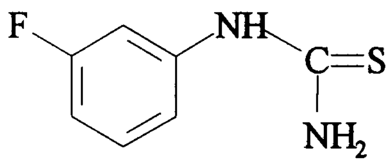

Eine Mischung aus 300 g (2,67 Mol) 3-Fluor-anilin, 271 g (2,67 Mol) konz. Salzsäure und 259 g (2,67 Mol) Kaliumthiocyanat wird 6 Stunden unter Rückfluß erhitzt. Nach dem Abkühlen wird das kristallin angefallene Produkt durch Absaugen isoliert.

Man erhält 190 g (41,5% der Theorie) N-(3-Fluor-phenyl)-thioharnstoff vom Schmelzpunkt 109 °C.

Stufe 2:

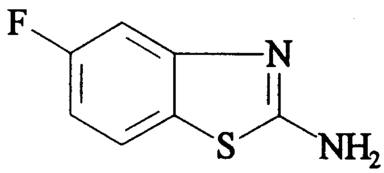

Eine Lösung von 176 g (1,1 Mol) Brom in 190 ml Chloroform wird unter Rühren tropfenweise zu einer auf 0 °C abgekühlten Lösung von 190 g (1,1 Mol) N-(3-Fluor-phenyl)-thioharnstoff in 1100 ml Chloroform gegeben und die Reaktionsmischung wird dann 4 Stunden unter Rückfluß erhitzt. Dann wird eingeengt, der Rückstand in 3 Liter Wasser aufgenommen und mit konz. Schwefelsäure auf pH = 1 eingestellt. Anschließend wird filtriert und das Filtrat mit konz. Ammoniak alkalisch gestellt. Das kristallin angefallene Produkt wird durch Absaugen isoliert.

Man erhält 147 g (70% der Theorie) 2-Amino-5-fluor-benzthiazol vom Schmelzpunkt 167 °C.

Stufe 3

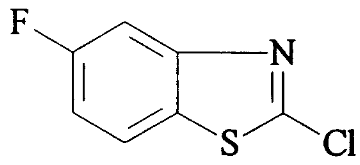

Eine Lösung von 242 g (3,5 Mol) Natriumnitrit in 440 ml Wasser wird unter Rühren tropfenweise zu einer auf -10 °C abgekühlten Mischung aus 147 g (0,87 Mol) 2-Amino-5-fluor-benzthiazol, 1700 ml konz. Salzsäure und 20 g Kupferpulver gegeben und die Reaktionsmischung wird 60 Minuten bei 0 °C und weiter 60 Minuten bei 50 °C gerührt. Dann wird mit Chloroform extrahiert und von der organischen Phase das Lösungsmittel im Wasserstrahlvakuum sorgfältig abdestilliert.

Man erhält 80 g (49% der Theorie) 2-Chlor-5-fluor-benzthiazol als kristallinen Rückstand vom Schmelzpunkt 69 °C.

Beispiel (II-2)

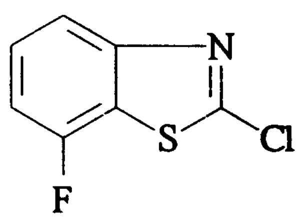

Stufe 1:

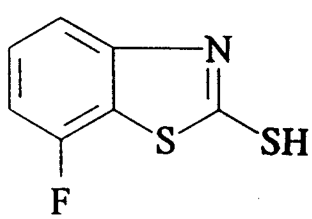

Eine Mischung aus 12,9 g (0,1 Mol) 2,3-Difluoranilin und 30 ml Dimethylformamid wird zum Rückfluß erhitzt und dabei wird eine Lösung von 48 g (0,3 Mol) Kalium-ethylxanthogenat in 80 ml Dimethylformamid innerhalb einer Stunde zugetropft. Durch Abnahme von Destillat wird die Sumpftemperatur bei 145 °C gehalten. Das Reaktionsgemisch wird 15 Stunden unter Rückfluß gerührt und nach Abkühlen in 1 Liter Wasser gegossen. Dann wird bei 10 °C mit einer Lösung von 20 ml konz. Salzsäure in 30 ml Wasser angesäuert und das kristalline Produkt durch Absaugen isoliert.

Man erhält 18,0 g (87% der Theorie) 2-Mercapto-7-fluor-benzthiazol vom Schmelzpunkt 193 °C.

Stufe 2

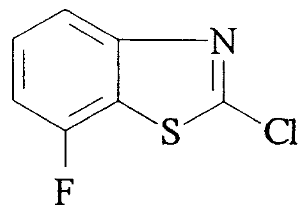

9,3 g (44,5 mMol) 2-Mercapto-7-fluor-benzthiazol werden zu 50 ml Thionylchlorid gegeben und nach Zusatz von 1 ml Dimethylformamid wird die Mischung langsam zum Rückfluß erhitzt. Nach einstündigem Rühren unter Rückfluß wird abgekühlt und im Wasserstrahlvakuum eingeengt. Der Rückstand wird in Methylenchlorid aufgenommen, mit Wasser gewaschen, mit Natriumsulfat getrocknet und filtriert. Das Filtrat wird eingeengt und der Rückstand wird durch Säulenchromatographie (Kieselgel/Methylenchlorid) gereinigt.

Man erhält 4,3 g (45% der Theorie) 2-Chlor-7-fluor-benzthiazol als gelbes Öl.

Beispiel (II-3)

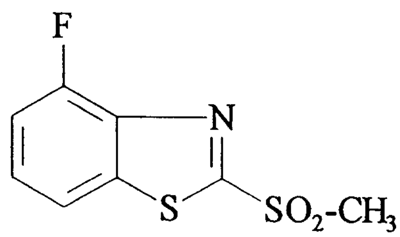

Stufe 1

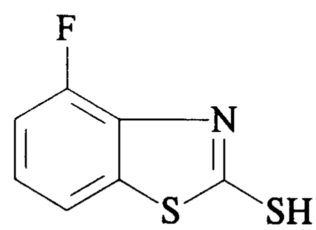

Eine Mischung aus 97 g (0,75 Mol) 2,6-Difluoranilin, 120 g (0,75 Mol) Kalium-ethylxanthogenat und 750 ml Dimethylsulfoxid wird 3 Stunden bei 120 °C gerührt. Nach Abkühlen wird mit 300 ml Wasser verdünnt und mit konz. Salzsaure auf pH = 1 gestellt. Das hierbei kristallin angefallene Produkt wird durch Absaugen isoliert.

Man erhält 120 g (80% der Theorie) 2-Mercapto-4-fluor-benzthiazol vom Schmelzpunkt 180 °C.

Stufe 2

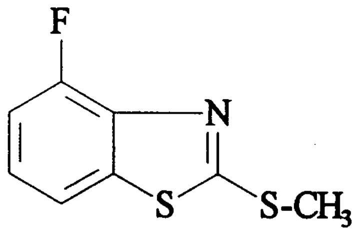

111 g (0,77 Mol) Methyliodid werden unter Rühren tropfenweise zu einer auf 0 °C abgekühlten Mischung aus 120 g (0,65 Mol) 2-Mercapto-4-fluor-benzthiazol, 26 g (0,65 Mol) Natriumhydroxid und 750 ml Wasser gegeben. Das Reaktionsgemisch wird 3 Stunden gerührt und das kristallin angefallene Produkt durch Absaugen isoliert.

Man erhält 51 g (39% der Theorie) 2-Methylthio-4-fluor-benzthiazol vom Schmelzpunkt 30 °C.

Stufe 3

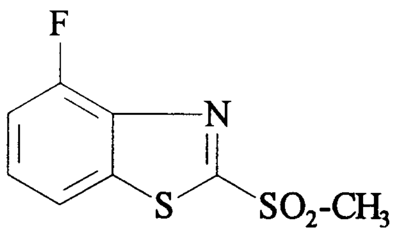

145 ml einer 35%igen wäßrigen Hydrogenperoxidlösung (1,7 Mol $H_2O_2$) werden unter Rühren tropfenweise zu einer auf 30 °C gehaltenen Mischung aus 51 g (0,25 Mol) 2-Methylthlo-4-fluor-benzthiazol, 500 ml Chloroform, 25 ml Ameisensäure und 2,5 ml konz. Schwefelsäure sowie 2 g Ammoniummolybdat gegeben. Das Reaktionsgemisch wird 2 Stunden bei 30 °C gerührt und das kristallin angefallene Produkt wird durch Absaugen isoliert.

Man erhält 31 g (54% der Theorie) 2-Methylsulfonyl-4-fluor-benzthiazol vom Schmelzpunkt 160 °C.

Analog Beispiel (II-3) erhält man über 2-Mercapto-7-fluor-benzthiazol und 2-Methylthio-7-fluor-benzthiazol beispielsweise auch 2-Methylsulfonyl-7-fluor benzthiazol der nachstehenden Formel

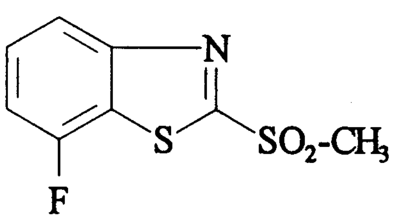

Anwendungsbeispiele:

In den nachfolgenden Anwendungsbeispielen wird die nachstehend aufgeführte Verbindung als Vergleichssubstanz herangezogen:

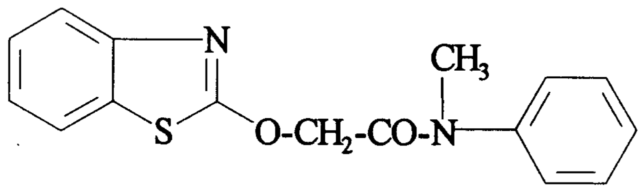

(A)

Benzthiazol-2-yl-oxy-essigsäure-N-methyl-anilid (Mefenacet)
(bekannt aus EP-A-5501, Bsp. 1)

Beispiel A: Pre-emergence-Wasseroberflächenbehandlung bei verpflanztem Wasserreis

Zur Herstellung einer applizierfähigen Zubereitung wird 1 Teil Wirkstoff mit 5 Teilen Aceton gelöst; anschließend wird 1 Teil Benzyloxy-polyglykol-ether als Emulgator zugegeben. Dann wird Wasser bis zur gewünschten Konzentration zugegeben. In Töpfe, die mit Erde gefüllt sind, wird Reis im 2-3-Blattstadium verpflanzt. Samen von Testpflanzen werden ausgelegt (1 cm tief). Zwei Tage später werden die Töpfe mit Wasser 3 cm überstaut. Anschließend werden die Wirkstoffzubereitungen auf die Wasseroberfläche ausgebracht. 4 Wochen später wird die herbizide Wirkung und die Schädigung der behandelten Pflanzen im Vergleich zu unbehandelten visuell in % ausgewertet. 0% bedeutet keine Wirkung, 100% bedeutet völliges Absterben.

In diesem Test zeigt sich die zum Teil sehr gute Verträglichkeit der erfindungsgemäßen Wirkstoffe - insbesondere der Verbindungen aus den Herstellungsbeispielen (3), (4), (5), (6) und (7) - in Reis bei im Vergleich mit der bekannten Verbindung (A) wesentlich stärkerer Wirkung gegen Unkräuter, insbesondere gegen Hühnerhirse (Echinochloa crus galli). Das gleiche gilt für die Verbindung von Herstellungsbeispiel (39).

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalem Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

In diesem Test zeigen beispielsweise die Verbindungen gemäß Herstellungsbeispiel (2), (4), (5), (6) und (7) bei guter Verträglichkeit gegenüber Kulturpflanzen, wie z.B. Soja, starke Wirkung gegen Unkräuter. Entsprechendes gilt auch für die Verbindung von Herstellungsbeispiel (39).

**Patentansprüche**

**1.** Fluorbenzthiazolyloxyacetamide der allgemeinen Formel (I),

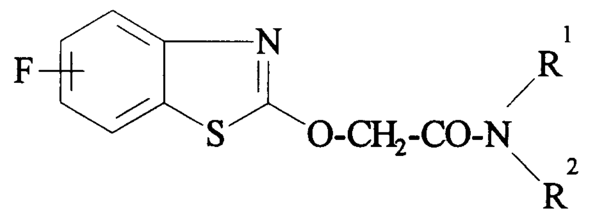

(I)

in welcher

$R^1$ für Wasserstoff oder für einen gegebenenfalls substituierten Rest aus der Reihe Alkyl, Alkenyl, Alkinyl oder Aralkyl steht,

$R^2$ für einen gegebenenfalls substituierten Rest aus der Reihe Alkyl, Alkenyl, Alkinyl, Cycloalkyl, Cycloalkenyl, Aralkyl, Aryl, Alkoxy, Alkenyloxy oder Alkinyloxy steht, oder

$R^1$ und $R^2$ zusammen mit dem Stickstoffatom, an welches sie gebunden sind, einen gegebenenfalls substituierten, gesättigten oder ungesättigten Stickstoff-Heterocyclus bilden, der weitere Heteroatome enthalten kann und an den eine Benzo-Gruppierung anelliert sein kann.

**2.** Verbindungen der Formel (I) gemäß Anspruch 1, dadurch gekennzeichnet, daß darin

$R^1$ für Wasserstoff, $C_1$-$C_8$-Alkyl (welches gegebenenfalls durch Fluor, Chlor, Cyano oder $C_1$-$C_4$-Alkoxy substituiert ist), für $C_2$-$C_8$-Alkenyl (welches gegebenenfalls durch Fluor und/oder Chlor substituiert ist), für $C_2$-$C_8$-Alkinyl oder für Benzyl steht,

$R^2$ für $C_1$-$C_8$-Alkyl (welches gegebenenfalls durch Fluor, Chlor, Cyano oder $C_1$-$C_4$-Alkoxy substituiert ist), $C_2$-$C_8$-Alkenyl (welches gegebenenfalls durch Fluor und/oder Chlor substituiert ist), für $C_2$-$C_8$-Alkinyl, für $C_3$-$C_6$-Cycloalkyl (welches gegebenenfalls durch Chlor und/oder $C_1$-$C_3$-Alkyl substituiert ist), für $C_5$- oder $C_6$-Cycloalkenyl, für Benzyl (welches gegebenenfalls durch Fluor, Chlor und/oder $C_1$-$C_4$-Alkyl substituiert ist), für Phenyl (welches gegebenenfalls durch Fluor, Chlor, Brom, Iod, Cyano, Nitro, $C_1$-$C_4$-Alkyl, Trifluormethyl, $C_1$-$C_4$-Alkoxy und/oder $C_1$-$C_4$-Alkylthio substituiert ist), für $C_1$-$C_8$-Alkoxy (welches gegebenenfalls durch $C_1$-$C_4$-Alkoxy substituiert ist), oder für $C_3$-$C_4$-Alkenyloxy steht, oder

$R^1$ und $R^2$ zusammen mit dem Stickstoffatom, an das sie gebunden sind, einen gegebenenfalls ein- bis dreilach durch $C_1$-$C_3$-Alkyl substituierten, gesättigten oder ungesättigten fünf- bis siebengliedrigen Stickstoffheterocyclus bilden, welcher gegebenenfalls benzanelliert ist.

**3.** Verbindungen der Formel (I) gemaß Anspruch 1, dadurch gekennzeichnet, daß darin

$R^1$ für $C_1$-$C_4$-Alkyl, Allyl oder Propargyl steht,

$R^2$ für $C_1$-$C_6$-Alkyl, $C_1$-$C_2$-Alkoxy-$C_1$-$C_2$-alkyl, Allyl, Propargyl, Cyclopentyl, Cyclohexyl, Cyclohexenyl, Benzyl, Phenyl (welches gegebenenfalls durch Fluor, Chlor, Brom, Cyano, Nitro, Methyl, Ethyl, Trifluormethyl, Methoxy oder Ethoxy substituiert ist), $C_1$-$C_6$-Alkoxy oder $C_1$-$C_2$-Alkoxy-$C_1$-$C_2$-alkoxy steht, oder

$R^1$ und $R^2$ zusammen mit dem Stickstoffatom, an das sie gebunden sind, für gegebenenfalls ein- bis dreifach durch Methyl und/oder Ethyl substituiertes Piperidinyl, für gegebenenfalls ein- oder zweifach durch Methyl und/oder Ethyl substituiertes Pyrrolidinyl, für Perhydroazepinyl oder für 1,2,3,4-Tetrahydro(iso)-chinolinyl stehen.

**4.** 4-Fluor-, 5-Fluor-, 6-Fluor- und 7-Fluor-benzthiazolyloxyacetamide der Formeln (IC), (ID), (IA) bzw. (IB) gemäß Ansprüchen 1 bis 3:

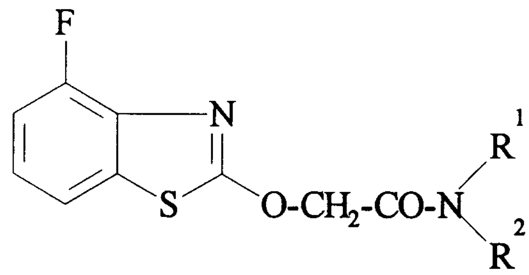

(IC),

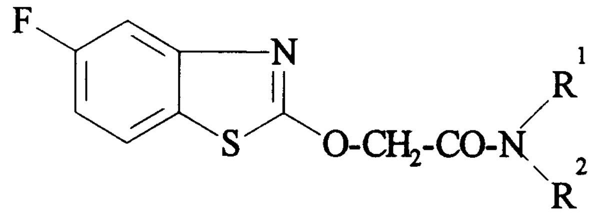

(ID),

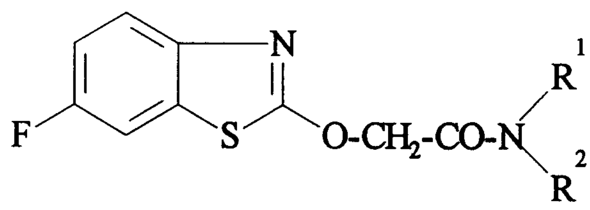

(IA),

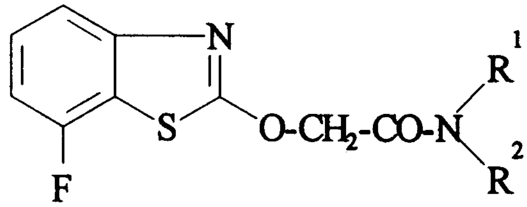

(IB).

**5.** Verfahren zur Herstellung von Fluorbenzthiazolyloxyacetamiden der Formel (I) gemäß Anspruch 1, dadurch gekennzeichnet, daß man Fluorbenzthiazole der Formel (II)

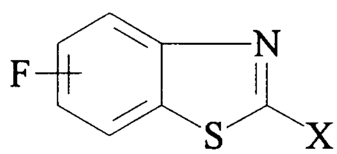

(II)

in welcher
X für Halogen oder Alkylsulfonyl steht,
mit Hydroxyacetamiden der allgemeinen Formel (III)

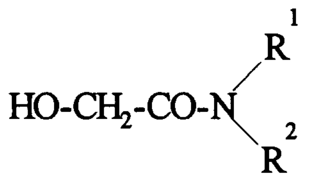

(III)

in welcher

$R^1$ und $R^2$ die oben angegebenen Bedeutungen haben,

gegebenenfalls in Gegenwart eines Verdünnungsmittels, gegebenenfalls in Gegenwart eines Säurebindemittels und gegebenenfalls in Gegenwart eines Katalysators umsetzt.

**6.** Herbizide Mittel, gekennzeichnet durch einen Gehalt an mindestens einer Verbindung der Formel (I) gemäß Anspruch 1.

**7.** Verwendung von Verbindungen der allgemeinen Formel (I) gemäß Anspruch 1 zur Bekämpfung von unerwünschtem Pflanzenwachstum.

**8.** Verfahren zur Bekämpfung von Unkräutern, dadurch gekennzeichnet, daß man Verbindungen der allgemeinen Formel (I) gemäß Anspruch 1 auf die Unkräuter oder ihren Lebensraum einwirken läßt.

**9.** Verfahren zur Herstellung von herbiziden Mittein, dadurch gekennzeichnet, daß man Verbindungen der allgemeinen Formel (I) gemäß Anspruch 1 mit Streckmitteln und/oder oberflächenaktiven Mitteln vermischt.

**10.** Fluorbenzthiazole der allgemeinen Formel (II)

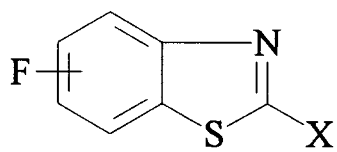

(II)

in welcher

X für Halogen oder Alkylsulfonyl steht,

ausgenommen die Verbindungen 2,6-Difluor-benzthiazol und 2-Chlor-6-fluor-benzthiazol.

Europäisches Patentamt

# EUROPÄISCHER RECHERCHENBERICHT

Nummer der Anmeldung
EP 93 11 4134

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| D,X | EP-A-0 005 501 (BAYER AKTIENGESELLSCHAFT)<br>* Ansprüche 1-6 *<br>--- | 1-3,5-9 | C07D277/68<br>A01N43/78<br>C07D277/76<br>C07D417/12 |
| A | EP-A-0 161 602 (BAYERAG)<br>* Ansprüche 1,5-9 *<br>--- | 1,6-9 | |
| A | EP-A-0 444 764 (SCHERING AKTIENGESELLSCHAFT)<br>* Ansprüche *<br>--- | 1,6-10 | |
| D,A | CHEMICAL ABSTRACTS, vol. 113, no. 3, 16. Juli 1990, Columbus, Ohio, US; abstract no. 23901k, Seite 662 ;<br>* Zusammenfassung *<br>& JP-A-0 204 784 (NIHON TOKUSHU NOYAKU SEIZO K.K.) 9. Januar 1990<br>----- | 1,6-10 | |

RECHERCHIERTE SACHGEBIETE (Int.Cl.5)

C07D

Der vorliegende Recherchenbericht wurde für alle Patentansprüche erstellt

| Recherchenort | Abschlußdatum der Recherche | Prüfer |
|---|---|---|
| DEN HAAG | 2. Dezember 1993 | Henry, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)